(19) Europäisches Patentamt
European Patent Office
Office européen des brevets

(11) EP 0 528 922 B1

(12) **EUROPÄISCHE PATENTSCHRIFT**

(45) Veröffentlichungstag und Bekanntmachung des Hinweises auf die Patenterteilung:
**16.06.1999 Patentblatt 1999/24**

(21) Anmeldenummer: **91909549.7**

(22) Anmeldetag: **14.05.1991**

(51) Int Cl.[6]: **C07D 471/04**, A61K 31/47
// (C07D471/04, 221:00, 221:00)

(86) Internationale Anmeldenummer:
**PCT/EP91/00895**

(87) Internationale Veröffentlichungsnummer:
**WO 91/17991 (28.11.1991 Gazette 1991/27)**

(54) **NEUE SULFONYLVERBINDUNGEN**

NEW SULFONYL COMPOUNDS

NOUVEAUX COMPOSES DE SULFONYLE

(84) Benannte Vertragsstaaten:
**AT BE CH DE DK ES FR GB GR IT LI LU NL SE**

(30) Priorität: **16.05.1990 CH 165890**

(43) Veröffentlichungstag der Anmeldung:
**03.03.1993 Patentblatt 1993/09**

(73) Patentinhaber: **BYK GULDEN LOMBERG CHEMISCHE FABRIK GMBH**
**D-78403 Konstanz (DE)**

(72) Erfinder:
- **FLOCKERZI, Dieter**
  **D-7753 Allensbach (DE)**
- **KLEMM, Kurt**
  **D-7753 Allensbach (DE)**
- **AMSCHLER, Hermann**
  **D-7760 Radolfzell (DE)**
- **FIGALA, Volker**
  **D-7753 Allensbach 4 (DE)**
- **BEUME, Rolf**
  **D-7750 Konstanz 18 (DE)**
- **HÄFNER, Dietrich**
  **D-7750 Konstanz (DE)**
- **HANAUER, Guido**
  **D-7750 Konstanz 19 (DE)**
- **ELTZE, Manfrid**
  **D-7750 Konstanz (DE)**
- **SCHUDT, Christian**
  **D-7750 Konstanz (DE)**
- **KILIAN, Ulrich**
  **D-7752 Reichenau 2 (DE)**

(56) Entgegenhaltungen:
**EP-A- 0 247 971** **FR-A- 2 259 611**
**US-A- 3 899 494**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 528 922 B1

## Beschreibung

Anwendungsgebiet der Erfindung

Die Erfindung betrifft neue Sulfonylverbindungen, Verfahren zu ihrer Herstellung, ihre Anwendung und sie enthaltende Arzneimittel. Die erfindungsgemäßen verbindungen werden in der pharmazeutischen Industrie zur Herstellung von Medikamenten verwendet.

Bekannter technischer Hintergrund

In der DE-OS 21 23 328 und im USP 3,899,494 werden substituierte Benzo-naphthyridine beschrieben, die sich durch eine ausgeprägte Blutplättchenaggregationshemmung auszeichnen. Die Anwendung der unter diese Schutzrechte fallenden Verbindung mit dem vorgeschlagenen INN Benafentrin als Bronchodilator und zur Behandlung entzündlicher Atemwegserkrankungen wird in der Europäischen Patentanmeldung 247 971 offenbart.

Beschreibung der Erfindung

Es wurde nun gefunden, daß die unten näher beschriebenen Verbindungen, die sich von der verbindung Benafentrin insbesondere durch die Sulfonylsubstitution anstelle der Acetylsubstitution an der Aminogruppe unterscheiden, überraschende und besonders vorteilhafte Eigenschaften besitzen.

Gegenstand der Erfindung sind somit in einem ersten Aspekt Verbindungen der Formel I

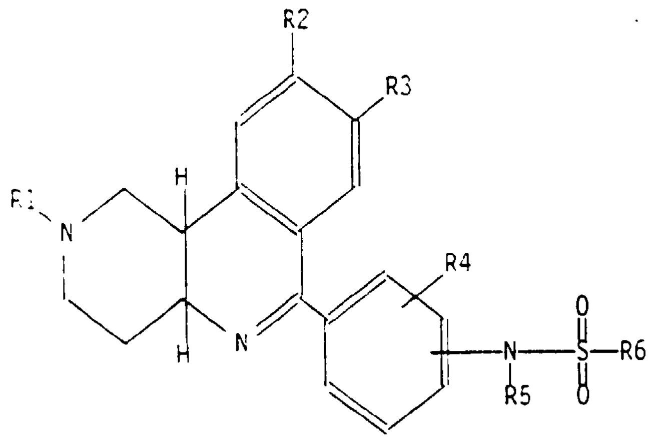

(I)

worin

R1 1-4C-Alkyl,
R2 Wasserstoff (H), 1-4C-Alkyl oder 1-4C-Alkoxy,
R3 Wasserstoff (H), 1-4C-Alkyl oder 1-4C-Alkoxy,
R4 Wasserstoff (H), Methyl oder Methoxy,
R5 Wasserstoff (H) oder 1-4C-Alkyl und
R6 1-4C-Alkyl, Phenyl oder substituiertes Phenyl mit einem oder zwei gleichen oder verschiedenen Substituenten aus der Gruppe 1-4C-Alkyl, 1-4C-Alkoxy, Hydroxy und Halogen bedeutet,

und ihre Salze.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und insbesondere der Methylrest.

1-4C-Alkoxyreste enthalten neben dem Sauerstoffatom einen der vorstehend genannten 1-4C-Alkylreste. Bevorzugt ist der Methoxyrest.

Halogen im Sinne der vorliegenden Erfindung ist Brom, Chlor und Fluor.

Als beispielhafte substituierte Phenylreste R6 seien genannt die Reste: 4-Methylphenyl, 4-tert.-Butylphenyl, 4-Methoxyphenyl, 4-Fluorphenyl, 4-Chlorphenyl, 2,5-Dichlorphenyl und 4-hydroxyphenyl.

Als Salze kommen für Verbindungen der Formel I bevorzugt alle Säureadditionssalze in Betracht. Besonders

erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat, 3-Hydroxy-2-naphthoat oder Mesilat.

Hervorzuheben sind solche Verbindungen der Formel I, in denen

R1 1-4C-Alkyl,
R2 1-4C-Alkoxy,
R3 1-4C-Alkoxy,
R4 Wasserstoff oder Methyl,
R5 Wasserstoff oder 1-4C-Alkyl,
R6 1-4C-Alkyl, Phenyl oder substituiertes Phenyl mit einem Substituenten aus der Gruppe 1-4C-Alkyl, 1-4C-Alkoxy und Halogen bedeutet,

und ihre Salze.

Besonders hervorzuheben sind solche Verbindungen der Formel 1, in denen

R1 Methyl,
R2 Methoxy,
R3 Methoxy und
R4 Wasserstoff oder Methyl bedeutet,

der Rest -N(R5)$SO_2$R6 in 4-Position an dem in 6-Stellung am Benzo-naphthyridinring gebundenen Phenylrest steht,

R5 Wasserstoff, Methyl oder Ethyl und
R6 Methyl, 4-Methylphenyl, 4-Methoxyphenyl oder 4-Fluorphenyl bedeutet,

und ihre Salze.

Der Benzo-naphthyridincyclus besitzt (an den Positionen 4a und 10b) zwei Chiralitätszentren. Die Erfindung umfaßt daher alle denkbaren Enantiomeren und Diastereomeren sowie die Racemate und Gemische davon. Bevorzugt sind jene Verbindungen der Formel I, in denen die Wasserstoffatome in den Positionen 4a und 10b cis-ständig sind.

Bevorzugt sind die enantiomer reinen, zueinander spiegelbildlichen Cis-Verbindungen, die das linear polarisierte Licht in (+)- bzw. (-)-Richtung drehen [(+)-Enantiomer und (-)-Enantiomer]. Die Trennung der trans-Verbindungen von den (dazu diastereomeren) Cis-Verbindungen erfolgt - ebenso wie die Trennung der (+)- und (-)-Enantiomeren - auf eine dem Fachmann vertraute Weise, z.B. so wie in der Europäischen Patentanmeldung 247 971 beschrieben.

Besonders bevorzugt sind in diesem Zusammenhang solche Verbindungen der Formel I, die sich von Verbindungen der Formel II herleiten, die die gleiche absolute Konfiguration haben wie die Verbindung (-)-cis-6-(4-Aminophenyl)-8,9-dimethoxy--1,2,3,4,4a,10b-hexahydro-2-methyl-benzo[c] [1,6]-naphthyridin mit dem optischen Drehwert $[\alpha]^{22}_{578Hg}$ = -213° (c=1, Chloroform).

Weiterer Gegenstand der Erfindung ist ein Verfahren zur Herstellung der erfindungsgemäßen Verbindungen der Formel I, und ihrer Salze. Das Verfahren ist dadurch gekennzeichnet, daß man

a) Verbindungen der Formel II,

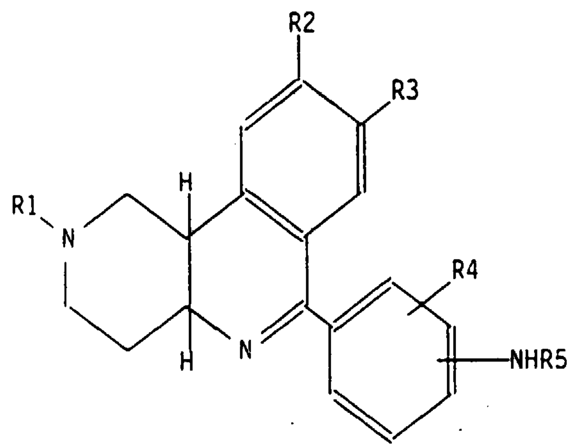

(II)

worin R1, R2, R3, R4 und R5 die oben angegebenen Bedeutungen haben, mit Sulfonylverbindungen der Formel III,

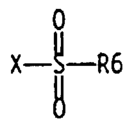

(III)

worin R6 die oben angegebene Bedeutung hat und X eine geeignete Abgangsgruppe darstellt, umsetzt, oder daß man

b) zur Herstellung von Verbindungen I, in denen R5 1-4C-Alkyl bedeutet, Verbindungen der Formel I, in denen R5 Wasserstoff bedeutet, mit einem Alkylierungsmittel der Formel IV,

Y-R5 (IV)

worin R5 1-4C-Alkyl und Y eine Abgangsgruppe bedeutet, alkyliert, oder daß man

c) Verbindungen der Formel V,

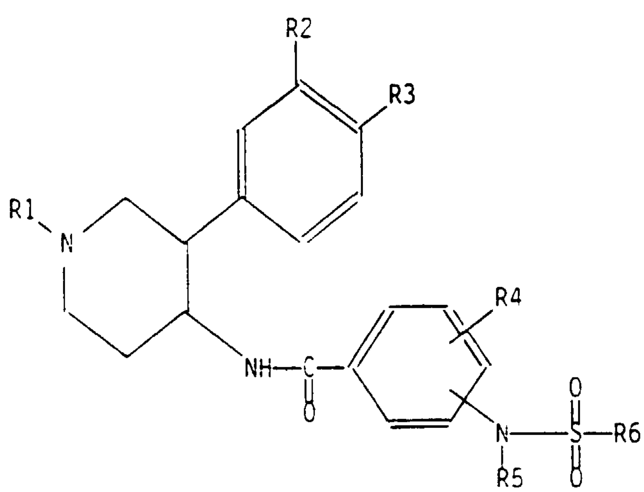

(V)

worin R1, R2, R3, R4, R5 und R6 die oben angegebenen Bedeutungen haben, cyclokondensiert und daß man gewünschtenfalls anschließend die nach a), b) oder c) erhaltenen Verbindungen I in ihre Salze überführt, oder daß man gewünschtenfalls anschließend aus erhaltenen Salzen der Verbindungen I die Verbindungen I freisetzt.

Die Umsetzung der Verbindungen II mit den Verbindungen III erfolgt in inerten Lösungsmitteln auf eine weise, wie sie dem Fachmann für die Herstellung von Sulfonamiden bekannt ist. Die Abgangsgruppe X ist bevorzugt ein Halogenatom, insbesondere ein Chloratom. Die Umsetzung erfolgt bevorzugt in Gegenwart einer Hilfsbase, z.B. eines organischen Amins, wie Triethylamin oder Pyridin, oder z.B. eines Carbonates, wie Kaliumcarbonat oder Natriumcarbonat.

Die N-Alkylierung gemäß Verfahrensvariante b) wird in einer dem Fachmann an sich vertrauten weise, gegebenenfalls unter Phasentransfer-Bedingungen, bevorzugt in Gegenwart geeigneter Basen oder nach vorheriger Deprotonierung der Verbindungen I mit R5 = Wasserstoff durchgeführt.

Als Deprotonierungsmittel kommen vor allem solche Agenzien in Frage, für die die Acidität des Protons am Stickstoff groß genug ist, um eine Anionbildung zu erzielen. Neben metallorganischen Verbindungen, wie z.B. Butyllithium, seien beispielsweise Metallhydride, insbesondere Natriumhydrid, oder Alkalialkoholate, z.B. Natriummethylat oder Kalium-tert.-butylat, oder Alkalihydroxide, z.B. Natriumhydroxid oder Kaliumhydroxid, oder Alkalicarbonate, z.B. Natriumcarbonat erwähnt.

Die Abgangsgruppe Y der Verbindungen IV ist eine Gruppe, die bei der Umsetzung von Y - R5 mit dem deprotonierten I leicht abgespalten wird, beispielsweise ein Halogenatom, wie Chlor, Brom oder Jod, oder die Alkylsulfatgruppe.

Die Deprotonierung und anschließende N-Alkylierung wird in inerten, wasserfreien Lösungsmitteln vorgenommen, wie sie für das Arbeiten mit starken Deprotonierungsmitteln geeignet sind, oder in Wasser-Lösungsmittelgemischen, wie sie beim Arbeiten unter Phasentransfer-Bedingungen eingesetzt werden. Beispielsweise seien offenkettige oder cyclische Ether, wie Diethylether, Dioxan oder Tetrahydrofuran, oder Lösungsmittel wie DMF oder DMSO genannt. Als wasser/Lösungsmittelmischungen seien beispielsweise die Mischungen von wasser mit Chloroform, Dichlormethan oder Benzol genannt. Die Umsetzung erfolgt bevorzugt unter schonenden Reaktionsbedingungen bei Temperaturen um oder unter 0°C.

Die Cyclokondensation gemäß Verfahrensvariante c) erfolgt auf eine dem Fachmann an sich bekannte Weise gemäß Bischler-Napieralski in Gegenwart eines geeigneten Kondensationsmittels, wie beispielsweise Polyphospnorsäure, Phosphorpentachlorid, Phosphorpentoxid oder bevorzugt Phosphoroxytrichlorid, in einem geeigneten inerten Lösungsmittel, z.B. in einem chlorierten Kohlenwasserstoff wie Chloroform, oder in einem cyclischen Kohlenwasserstoff wie Toluol oder Xylol, oder einem sonstigen inerten Lösungsmittel wie Acetonitril, oder ohne weiteres Lösungsmittel unter Verwendung eines Überschusses an Kondensationsmittel, vorzugsweise bei erhöhter Temperatur, insbesondere bei der Siedetemperatur des verwendeten Lösungs- bzw. Kondensationsmittels.

Die Verbindungen der Formel II mit R5 = Wasserstoff sind aus der DE-OS 21 23 328, dem USP 3,899,494 oder der EP-A-247 971 bekannt bzw. sie können auf analoge Weise, wie in den vorstehenden Schriften beschrieben, hergestellt werden. Verbindungen II mit R5 = 1-4C-Alkyl können aus den Verbindungen II mit R5 = Wasserstoff durch Alkylierung auf eine dem Fachmann vertraute Weise hergestellt werden.

Die Verbindungen der Formel V erhält man in an sich bekannter weise gemäß folgender Reaktionsgleichung aus den Verbindungen VI und VII

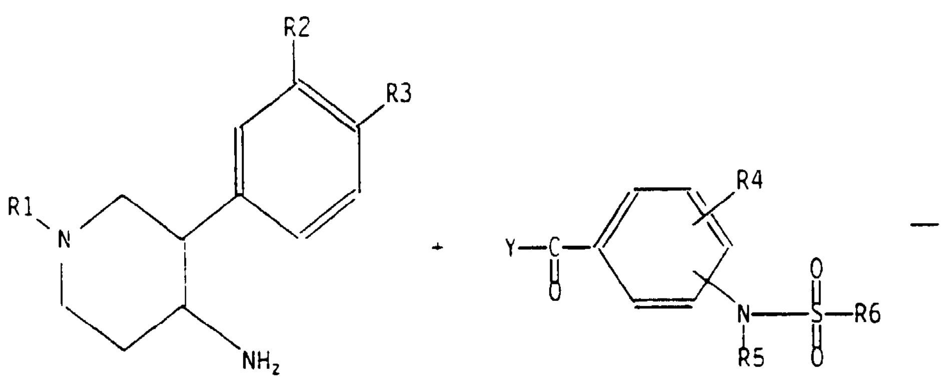

(V)

(VI) (VII)

worin R1, R2, R3, R4, R5 und R6 die oben angegebenen Bedeutungen haben und Y eine geeignete Abgangsgruppe, beispielsweise ein Chloratom darstellt.

Die Verbindungen VI sind z.B. aus der DE-OS 21 23 328 oder der EP-A-247 971 bekannt oder sie können auf analoge Weise hergestellt werden.

Die Verbindungen VII sind ebenfalls bekannt oder auf eine dem Fachmann bekannte weise herstellbar.

Zur Herstellung der enantiomer reinen Verbindungen I erfolgt die Trennung der trans-Verbindungen von den cis-Verbindungen sowie die Trennung der (+)- und (-)-Enantiomeren vorteilhafterweise auf der Stufe der Verbindungen II, die aus der EP-A-247 971 bekannt sind bzw. die wie dort beschrieben hergestellt und getrennt werden können.
Die folgenden Beispiele erläutern die Erfindung näher, ohne sie einzuschränken. Die in den Beispielen namentlich aufgeführten Verbindungen der allgemeinen Formel I sowie die Salze dieser Verbindungen sind bevorzugter Gegenstand der Erfindung. Schmp. bedeutet Schmelzpunkt, für Stunde(n) wird die Abkürzung h und für Minuten die Abkürzung Min. verwendet. Zers. steht für Zersetzung. Unter "Ether" wird Diethylether verstanden.

## Beispiele

1. rac-cis-8,9-Dimethoxy-1,2,3,4,4a,10b-hexahydro-2-methyl-6-(4-methylsulfonamidophenyl)-benzo[c][1,6]naphthyridin

Zur Lösung von 2,1 g rac-cis-6-(4-Aminophenyl)-8,9-dimethoxy-1,2,3,4,4a,10b-hexahydro-2-methyl-benzo[c][1,6]naphthyridin in 20 ml absolutem Pyridin tropft man eine Lösung von 0,6 ml Methansulfonsäurechlorid in 3 ml absolutem Dioxan und rührt anschließend das Gemisch noch 3 h bei 60 °C. Nach dem Abkühlen gießt man den Ansatz auf 100 ml Eis/Wassergemisch, alkalisiert mit verdünnter Natronlauge und extrahiert mit n-Butanol. Nach dem Abdampfen des n-Butanols wird der verbleibende Rückstand mit Dichlormethan extrahiert und die organische Phase über Natriumsulfat getrocknet und anschließend eingeengt. Man erhält als Rückstand 2,4 g der Titelverbindung, die mit etherischer HCl ins Dihydrochlorid überführt und aus nicht getrocknetem Methanol umkristallisiert wird. Ausbeute: 2,3 g der Titelverbindung als Dihydrochlorid-hydrat. Schmp. 239-240°C (Zersetzung).

2. rac-cis-8,9-Dimethoxy-1,2,3,4,4a,10b-hexahydro-2-methyl-6-[4-(p-toluolsulfonamido)-phenyl]-benzo[c] [1,6]naphthyridin

Analog Beispiel 1 erhält man die Titelverbindung, wenn p-Toluolsulfonsäurechlorid anstelle von Methansulfonsäurechlorid eingesetzt wird. Schmp. 219-221° C (Dihydrochlorid-hydrat).

3. rac-cis-8,9-Dimethoxy-1,2,3,4,4a,10b-hexahydro-2-methyl-6-[4-methyl-3-(p-toluolsulfonamido)-phenyl]-benzo[c] [1,6]naphthyridin

3,9 g rac-cis-3-(3,4-Dimethoxyphenyl)-1-methyl-4-[4-methyl-3-(p-toluolsulfonamido)-benzamido]-piperidin werden in 50 ml Phosphoroxytrichlorid 3 h unter Rückfluß zum Sieden erhitzt. Nach Abdestillieren des überschüssigen Phosphoroxytrichlorid verteilt man den Rückstand zwischen Dichlormethan und 2N Natronlauge, wäscht die organische Phase mit Wasser und trocknet sie über Natriumsulfat. Nach dem Abdestillieren des Dichlormethans reinigt man den Rückstand durch Kieselgelchromatographie. Die separierte Hauptproduktfraktion wird eingeengt und der feste Rückstand in Essigsäureethylester/Petrolether umkristallisiert. Man erhält 2,6 g der Titelverbindung als gelbliche Kristalle vom Schmp. 206-210°C (Zers.).
Die Ausgangsverbindung rac-cis-3-(3,4-Dimethoxyphenyl)-1-methyl-4-[4-methyl-3-(p-toluolsulfonamido)-benzamido]-piperidin erhält man durch Umsetzung von 4,6 g 4-Methyl-3-(p-toluolsulfonamido)-benzoesäurechlorid mit 3,0 g rac-cis-4-Amino-3-(3,4-dimethoxyphenyl)-1-methylpiperidin und 4 ml Triethylamin in 50 ml wasserfreiem Dichlormethan. Nach Ausschütteln mit $NaHCO_3$-Lösung, Trocknen der organischen Phase über Natriumsulfat und Einengen kristallisiert man den Rückstand in Methanol um. Ausbeute: 4,2 g, Schmp. 142-146 °C.

4. rac-cis-8,9-Dimethoxy-1,2,3,4,4d,10b-hexahydro-2-methyl-6-[2-(p-toluol-sulfonamido)-phenyl]-benzo[c] [1,6]naphthyridin

Analog Beispiel 3 erhält man die Titelverbindung aus 4,5 g rac-cis-3-(3,4-Dimethoxyphenyl)-1-methyl-4-[2-(p-toluolsulfonamido)-benzamido]-piperidin und 30 ml Phosphoroxytrichlorid, Ausbeute: 3,3 g bräunliche Kristalle.
Die Ausgangsverbindung rac-cis-3-(3,4'-Dimethoxyphenyl)-1-methyl-4-[2-(p-toluolsulfonamido)-benzamido]-piperidin erhält man analog Beispiel 3 aus 2-(p-Toluolsulfonamido)-benzoesäurechlorid und dem entsprechenden Piperidin. Ausbeute: 70 .

5. rac-cis-8,9-Dimethoxy-1,2,3,4,4a,10b-hexahydro-2-methyl-6-[4-(p-methoxy-phenylsulfonamido)-phenyl]-benzo[c] [1,6]naphthyridin

Analog Beispiel 1 erhält man die Titelverbindung, wenn p-Methoxyphenylsulfonsäurechlorid eingesetzt wird. Ausbeute: 66 %, Schmp. 210-217 °C (als Carbonathydrat aus Methanol kristallisiert).

Alternativ erhält man die Titelverbindung analog Beispiel 3, wenn rac-cis-3-[3,4-Dimethoxyphenyl)-1-methyl-4-[(4-p-methoxyphenylsulfonamido)-benzamido]-piperidin cyclokondensiert wird. Diese Ausgangsverbindung erhält man analog Beispiel 3, wenn 4-(p-Methoxyphenylsulfonamido)-benzoesäurechlorid eingesetzt wird.

6. <u>rac-cis-8,9-Dimethoxy-1,2,3,4,4a,10b-hexahydro-2-methyl-6-[4-(p-fluorphenylsulfonamido)-phenyl]-benzo[c][1,6]naphthyridin</u>

Analog Beispiel 3 erhält man die Titelverbindung aus rac-cis-3-(3,4-Dimethoxyphenyl)-1-methyl-4-[(p-fluorphenylsulfonamido)-benzamido]-piperidin eingesetzt wird. Ausbeute: 69 %, Schmp. 163-165 °C.

Die Ausgangsverbindung ist analog Beispiel 3 aus 4-(p-Fluorphenylsulfonamido)-benzoesäurechlorid erhältlich. Ausbeute: 60 %, Schmp. 108-113 °C.

7. <u>rac-cis-8,9-Dimethoxy-1,2,3,4,4a,10b-hexahydro-2-methyl-6-[4-(p-toluolsulfon-N-methylamido)-phenyl]-benzo[c][1,6]naphthyridin</u>

Analog Beispiel 3 erhält man die Titelverbindung aus rac-cis-3-(3,4-Dimethoxyphenyl)-1-methyl-4-[(p-toluolsulfon-N-methylamido)-benzamido]-piperidin; Ausbeute: 53 %, Schmp. 157-158°C (aus Methanol/Ether).

Die Ausgangsverbindung ist analog Beispiel 3 aus 4-(p-Toluolsulfon-N-methylamido)-benzoesäurechlorid erhältlich.

8. <u>rac-cis-8,9-Dimethoxy-1,2,3,4,4a,10b-hexahydro-2-methyl-6-[4-(p-toluolsulfon-N-ethylamido)-phenyl]-benzo[c][1,6]naphthyridin</u>

Analog dem vorstehenden Beispiel erhält man die Titelverbindung, wenn statt der N-methyl- die entsprechenden N-ethyl-Verbindungen eingesetzt werden. Ausbeute: 71 , Schmp. 160-166°C (Carbonat).

9. <u>(-)-cis-8,9-Dimethoxy-1,2,3,4,4a,10b-hexahydro-2-methyl-6-[4-(p-toluol-sulfonamido)-phenyl]-benzo[c][1,6]naphthyridin</u>

Analog Beispiel 1 wird die Titelverbindung erhalten, wenn (-)-cis-6-(4-Aminophenyl)-8,9-Dimethoxy-1,2,3,4,4a,10b-hexahydro-2-methyl-benzo[c][1,6]naphthyridin $[\alpha]^{22}_{578Hg}$ = -213° (c=1, $CHCl_3$) mit p-Toluolsulfonsäurechlorid umgesetzt wird. Ausbeute 82 %, Schmp. 178-183°C (gelbliche Kristalle aus Essigsäureethylester/Methanol); $[\alpha]^{22}_{D}$ = -81,6°, $[\alpha]^{22}_{578Hg}$ = -81,1°, (je c=1, Methanol).

10. <u>(+)-cis-8,9-Dimethoxy-1,2,3,4,4a,10b-hexahydro-2-methyl-6-[4-(p-toluolsulfonamido)-phenyl] -benzo[c] [1,6]naphthyridin</u>

Die Titelverbindung wird analog Beispiel 9 erhalten, wenn (+)-cis-6-(4-Aminophenyl)-8,9-dimethoxy-1,2,3,4,4a,10b-hexahydro-2-methyl-benzo[c][1,6]naphthyridin $[\alpha]^{22}_{578Hg}$ = +210° (c=1, $CHCl_3$) eingesetzt wird. Ausbeute: 81 %, Schmp. 180-181°C (gelbliche Kristalle aus Essigsäureethylester/Methanol); $[\alpha]^{22}_{D}$ = +84,2° (c=1, Methanol).

Die Ausgangsverbindungen (+)-cis- unc (-)-cis-6-(4-Aminophenyl)-8,9-dimethoxy-1,2,3,4,4a,10b-hexahydro-2-methyl-benzo[c][1,6]naphthyridin sind bekannt aus EP-A-247 971.

<u>Gewerbliche Anwendbarkeit</u>

Die erfindungsgemäßen Sulfonylverbindungen besitzen wertvolle pharmakologische Eigenschaften, die sie gewerblich verwertbar machen. Sie zeichnen sich vor allem durch solche Eigenschaften aus, die sie für die Therapie von Atemwegserkrankungen verschiedener Genese geeignet erscheinen lassen. Insbesondere können entzündliche und allergeninduzierte Bronchialerkrankungen aufgrund der antiinflammatorischen und broncholytischen Wirksamkeit der erfindungsgemäßen Verbindungen behandelt werden. Dabei zeichnen sich die erfindungsgemäßen Verbindungen durch eine sehr geringe Toxizität, eine große therapeutische Breite, eine langanhaltende Wirkung und das Fehlen wesentlicher Nebenwirkungen aus. Daneben besitzen die erfindungsgemäßen Sulfonylverbindungen blutdrucksenkende Eigenschaften.

Die broncholytische und antiinflammatorische Wirksamkeit der erfindungsgemäßen Sulfonylverbindungen ermöglicht ihren Einsatz in der Human- und Veterinärmedizin, wobei sie zur Behandlung und Prophylaxe von Krankheiten, die auf Erkrankungen der Bronchien beruhen, verwendet werden. Beispielsweise können akute und chronisch obstruktive Atemwegserkrankungen verschiedener Genese (Bronchitis, allergische Bronchitis, Asthma bronchiale) bei Mensch und Tier behandelt werden. Aufgrund der blutdrucksenkenden Wirksamkeit können die erfindungsgemäßen Verbindungen auch zur Behandlung von Bluthochdruckerkrankungen verschiedener Genese und den damit zusammenhängenden Begleiterkrankungen verwendet werden.

Ein weiterer Gegenstand der Erfindung ist daher ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen und/oder ihre pharmakologisch verträglichen Salze enthalten, Gegenstand der Erfindung.

Die erfindungsgemäßen Arzneimittel werden nach an sich bekannten Verfahren hergestellt, wobei bezüglich der Zubereitungen, Dosierungen, Darreichungsformen etc. beispielsweise auf die Ausführungen im Europäischen Patent 163 965 verwiesen wird. Von besonderer Bedeutung bei der Behandlung von Bronchialerkrankungen ist in diesem Zusammenhang die inhalative Verabreichung, für welche die erfindungsgemäßen Verbindungen aufgrund ihres Wirkungsprofiles hervorragend geeignet erscheinen.

## Pharmakologie

Die bronchospasmolytische Wirkung der erfindungsgemäßen Verbindungen kann an verschiedenen in vitro- und in vivo-Modellen nachgewiesen werden. In der folgenden Tabelle sind die untersuchten Substanzen mit Nummern versehen worden, die den Nummern der Substanzen in den Beispielen entsprechen.

### Bronchospasmolytische Wirkung nach i.t. Instillation am Modell des narkotisierten Meerschweinchens

Die erfindungsgemäßen Verbindungen wurden auf ihre bronchospasmolytische Wirkung nach i.t. Instillation am Modell des narkotisierten Meerschweinchens wie folgt näher untersucht:

**Tiere:**

Männliche Meerschweinchen, Dunkin-Hartley, Charles-River/Wiga, Gewicht 300-450 g, N=6 Tiere pro Versuchsgruppe.

**Methode:**

Urethan-Narkose; Präparation der V. jugularis zur i.v. Histaminapplikation; Einbringen eines Y-förmigen Trachealkatheters zur Messung des Flows und zur i.t. Applikation der Testsubstanz; Einbringen eines stumpfen, kurzen Pleuralkatheters zur Messung des Pleuraldrucks; Bestimmung der Lungenfunktionsparameter Compliance und Conductance (= 1/Resistance) mittels Buxco-Lungenfunktionsanalysator; PC-gestützte Erfassung der Meßdaten.

Auslösung von Histamin-induzierten Bronchospasmen 20 und 10 Min. vor sowie 2, 10, 30 und 60 Min. nach Substanzapplikation; für Compliance und Conductance werden die baseline-Werte vor jedem Bronchospasmus und die delta % Änderungen während des Bronchospasmus mittels PC-Programm erfaßt (T=4 sec); diese Daten bilden die Basis für die spätere Auswertung.

Provokationslösung: Histamin 4 bzw. 5 µg/kg (= 22 bzw. 27 nmol/kg) i.v. gelöst in 0,9 % NaCl-Lösung, Applikationsvolumen 1 ml/kg, Bolusinjektion; Einschlußkriterium: Compliance-Abnahme 70-90 %. Die Testsubstanzen werden, soweit sie nicht wasserlöslich sind, naßvermahlen und in einer Dosis von 3 µmol/kg bei einem Applikationsvolumen von 0,1 ml/kg i.t. verabreicht. Der applikationsfertigen Suspension wird 1 % Tween 80 zugegeben. Als Placebolösung wird bei gleicher Applikationsweise und gleichem Applikationsvolumen eine substanzfreie 10 % Succinylgelatine/aqua dest.-Lösung mit 1 % Tween 80 verwendet.

**Ergebnis:**

In der folgenden Tabelle I ist die prozentuale Hemmung der Histamin-induzierten Bronchokonstriktion nach i.t. Gabe von 3 µmol/kg Substanz im Vergleich zur Placebogruppe wiedergegeben. Errechnet wurde die mittlere, prozentuale bronchospasmolytische Wirkung über 60 Min. (Wirkg. 0-60), die maximale, prozentuale bronchospasmolytische Wirkung (Wirkg. max) und die prozentuale bronchospasmolytische Wirkung zum Zeitpunkt 60 Min. post appl.

(Wirkg. 60) jeweils im Vergleich mit dem korrespondierenden Placebo.

Tabelle 1

| Prozentuale Hemmung der Histamin-induzierten Bronchokonstriktion nach i.t. Gabe von 3 µmol/kg Substanz im Vergleich zur Placebogruppe | | | | | | |
|---|---|---|---|---|---|---|
| | Conductance | | | Compliance | | |
| Substanz Nr. | Wirkg. 0-60 | Wirkg. max | Wirkg. 60 | Wirkg. 0-60 | Wirkg. max | Wirkg. 60 |
| 1 | 55 % | 61 % | 51 % | 34 % | 37 % | 35 % |
| 2 | 61 % | 84 % | 60 % | 47 % | 72 % | 40 % |
| 9 | 57 % | 71 % | 51 % | 56 % | 70 % | 45 % |

**Patentansprüche**

1. Verbindungen der Formel I

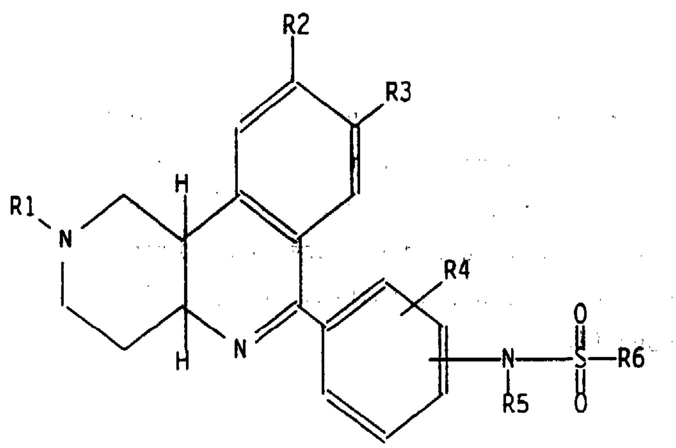

(I)

worin

R1 1-4C-Alkyl,
R2 Wasserstoff (H), 1-4C-Alkyl oder 1-4C-Alkoxy,
R3 Wasserstoff (H), 1-4C-Alkyl oder 1-4C-Alkoxy,
R4 Wasserstoff (H), Methyl oder Methoxy,
R5 Wasserstoff (H) oder 1-4C-Alkyl und
R6 1-4C-Alkyl, Phenyl oder substituiertes Phenyl mit einem oder zwei gleichen oder verschiedenen Substituenten aus der Gruppe 1-4C-Alkyl, 1-4C-Alkoxy, Hydroxy und Halogen bedeutet,

und ihre Salze.

2. Verbindungen der Formel I nach Anspruch 1, in denen

R1 1-4C-Alkyl,
R2 1-4C-Alkoxy,
R3 1-4C-Alkoxy,
R4 Wasserstoff oder Methyl,
R5 Wasserstoff oder 1-4C-Alkyl,
R6 1-4C-Alkyl, Phenyl oder substituiertes Phenyl mit einem Substituenten aus der Gruppe 1-4C-Alkyl, 1-4C-Alkoxy und Halogen bedeutet,

und ihre Salze.

3. Verbindungen der Formel I nach Anspruch 1, in denen

R1 Methyl,
R2 Methoxy,
R3 Methoxy und
R4 Wasserstoff oder Methyl bedeutet,

der Rest -N(R5)$SO_2$R6 in 4-Position an dem in 6-Stellung am Benzo-naphthyridinring gebundenen Phenylrest steht,

R5 Wasserstoff, Methyl oder Ethyl und
R6 Methyl, 4-Methylphenyl, 4-Methoxyphenyl oder 4-Fluorphenyl bedeutet,

und ihre Salze.

4. rac-cis-8,9-Dimethoxy-1,2,3,4,4a,10b-hexahydro-6-(4-methylsulfonamidophenyl)-2-methylbenzo[c] [1,6]naphthyridin und seine Salze.

5. rac-cis-8,9-Dimethoxy-1,2,3,4,4a,10b-hexahydro-2-methyl-6-[4-(p-toluolsulfonamido)-phenyl]-benzo[c] [1,6] naphthyridin und seine Salze.

6. (-)-cis-8,9-Dimethoxy-1,2,3,4,4a,10b-hexahydro-2-methyl-6-[4-(p-toluolsulfonamido)-phenyl]-benzo[c] [1,6]naphthyridin und seine Salze.

7. Verfahren zur Herstellung von Verbindungen der Formel I nach Anspruch 1, und ihren Salzen, dadurch gekennzeichnet, daß man

a) Verbindungen der Formel II,

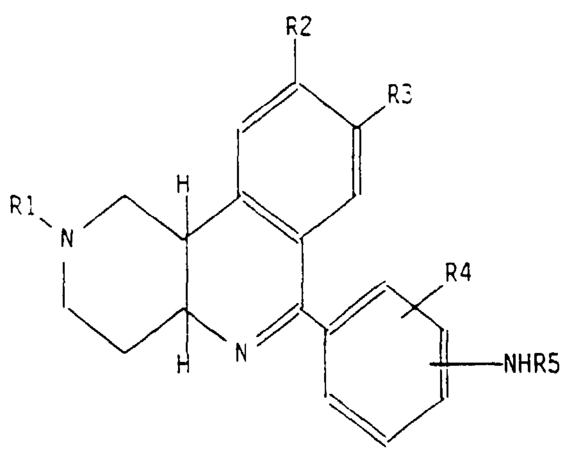

(II)

worin R1, R2, R3, R4 und R5 die in Anspruch 1 angegebenen Bedeutungen haben, mit Sulfonylverbindungen der Formel III,

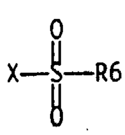

(III)

worin R6 die in Anspruch 1 angegebene Bedeutung hat und X eine geeignete Abgangsgruppe darstellt, umsetzt, oder daß man

b) zur Herstellung von Verbindungen I, in denen R5 1-4C-Alkyl bedeutet, Verbindungen der Formel I, in denen R5 Wasserstoff bedeutet, mit einem Alkylierungsmittel der Formel IV,

Y-R5 (IV)

worin R5 1-4C-Alkyl und Y eine Abgängsgruppe bedeutet, alkyliert, oder daß man

c) Verbindungen der Formel V,

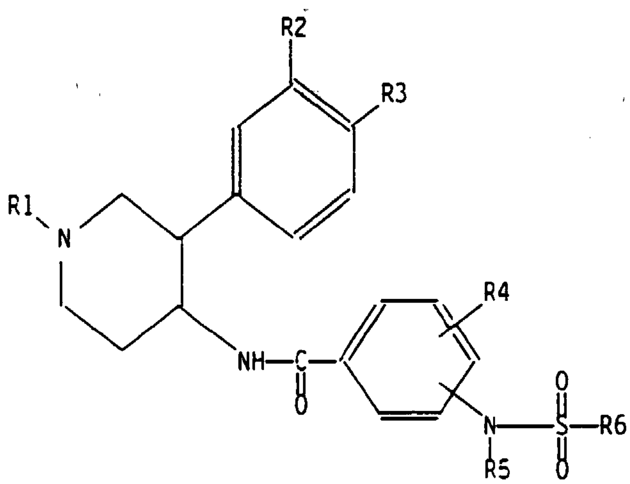

(V)

worin R1, R2, R3, R4, R5 und R6 die oben angegebenen Bedeutungen haben, cyclokondensiert und daß man gewünschtenfalls anschließend die nach a), b) oder c) erhaltenen Verbindungen I in ihre Salze überführt, oder daß man gewünschtenfalls anschließend aus erhaltenen Salzen der Verbindungen I die Verbindungen I freisetzt.

**8.** Arzneimittel enthaltend eine oder mehrere Verbindungen nach einem der Ansprüche 1, 2, 3, 4, 5 oder 6 und/oder ihre pharmakologisch verträglichen Salze.

**9.** Verbindungen nach einem der Ansprüche 1, 2, 3, 4, 5 oder 6 und/oder ihre pharmakologisch verträglichen Salze zur Anwendung bei der Behandlung und/oder Prophylaxe von Erkrankungen der Bronchien.

**10.** Verwendung der Verbindungen nach einem der Ansprüche 1, 2, 3, 4, 5 oder 6 und/oder ihrer pharmakologisch verträglichen Salze zur Herstellung von Arzneimitteln zur Behandlung und/oder Prophylaxe von Erkrankungen der Bronchien.

## Claims

**1.** Compounds of the formula I

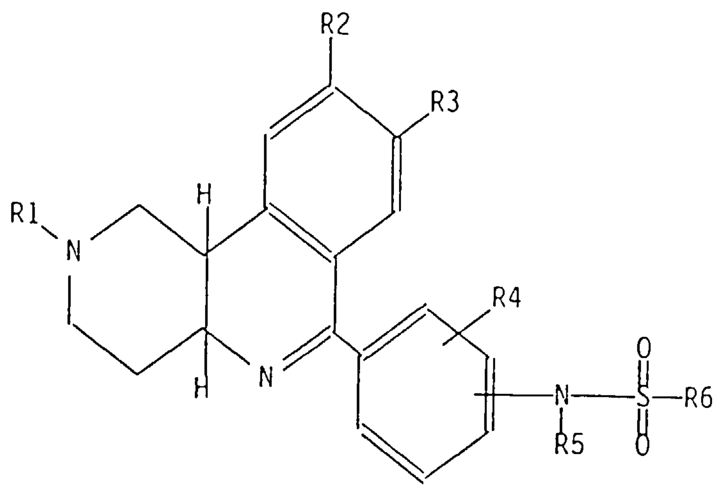

(I)

wherein

R1 denotes 1-4C-alkyl,
R2 denotes hydrogen (H), 1-4C-alkyl or 1-4C-alkoxy,
R3 denotes hydrogen (H), 1-4C-alkyl or 1-4C-alkoxy,
R4 denotes hydrogen (H), methyl or methoxy,
R5 denotes hydrogen (H) or 1-4C-alkyl and
R6 denotes 1-4C-alkyl, phenyl or substituted phenyl with one or two identical or different substituents from the group comprising 1-4C-alkyl, 1-4C-alkoxy, hydroxyl and halogen,

and their salts.

**2.** Compounds of the formula I according to Claim 1, in which

R1 denotes 1-4C-alkyl,
R2 denotes 1-4C-alkoxy,
R3 denotes 1-4C-alkoxy,
R4 denotes hydrogen or methyl,
R5 denotes hydrogen or 1-4C-alkyl and
R6 denotes 1-4C-alkyl, phenyl or substituted phenyl with one substituent from the group comprising 1-4C-alkyl, 1-4C-alkoxy and halogen,

and their salts.

**3.** Compounds of the formula I according to Claim 1, in which

R1 denotes methyl,
R2 denotes methoxy,
R3 denotes methoxy and
R4 denotes hydrogen or methyl,

the radical -N(R5)$SO_2$R6 is in the 4-position of the phenyl radical bonded in the 6-position on the benzo-naphthyridine ring,

R5 denotes hydrogen, methyl or ethyl and
R6 denotes methyl, 4-methylphenyl, 4-methoxyphenyl or 4-fluorophenyl,

and their salts.

**4.** rac-cis-8,9-Dimethoxy-1,2,3,4,4a,10b-hexahydro-6-(4-methylsulphonamidophenyl)-2-methylbenzo[c][1,6]naphthyridine and its salts.

5. rac-cis-8,9-Dimethoxy-1,2,3,4,4a,10b-hexahydro-2-methyl-6-[4-(p-toluenesulphonamido)-phenyl]-benzo[c][1,6] naphthyridine and its salts.

6. (-)-cis-8,9-Dimethoxy-1,2,3,4,4a,10b-hexahydro-2-methyl-6-[4-(p-toluenesulphonamido)-phenyl]-benzo[c][1,6] naphthyridine and its salts.

7. Process for the preparation of the compounds of the formula I according to Claim 1 and their salts, characterized in that

a) compounds of the formula II

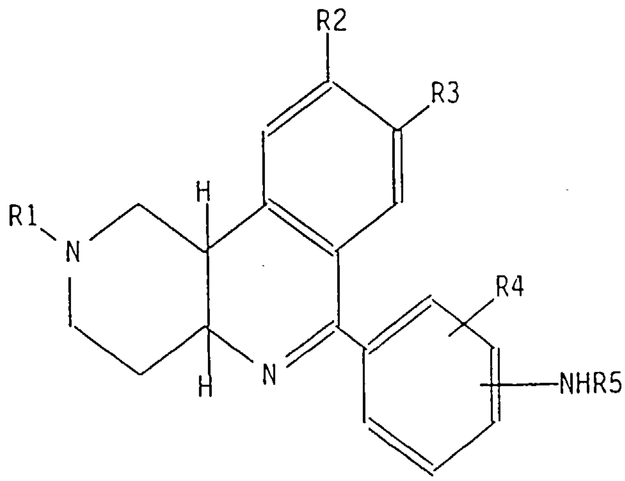

(II)

wherein R1, R2, R3, R4 and R5 have the meanings given in Claim 1, are reacted with sulphonyl compounds of the formula III

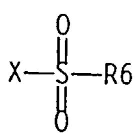

(III)

wherein R6 has the meaning given in Claim 1 and X represents a suitable leaving group, or in that

b) to prepare compounds I in which R5 denotes 1-4C-alkyl, compounds of the formula I in which R5 denotes hydrogen are alkylated with an alkylating agent of the formula IV

$$Y—R5 \qquad (IV)$$

wherein R5 denotes 1-4C-alkyl and Y denotes a leaving group, or in that

c) compounds of the formula V

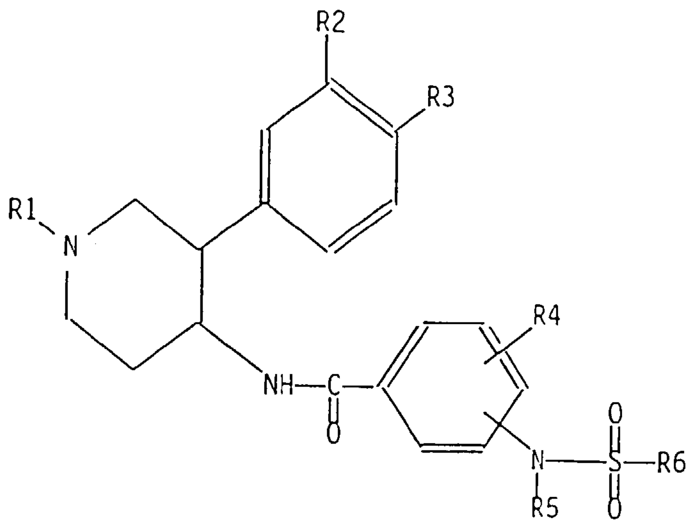

(V)

wherein R1, R2, R3, R4, R5 and R6 have the abovementioned meanings, are subjected to a cyclocondensation reaction, and in that, if desired, the compounds I obtained according to a), b) or c) are then converted into their salts, or in that, if desired, the compounds I are then liberated from resulting salts of the compounds I.

8. Medicaments containing one or more compounds according to one of Claims 1, 2, 3, 4, 5 or 6 and/or their pharmacologically tolerated salts.

9. Compounds according to one of Claims 1, 2, 3, 4, 5 or 6 and/or their pharmacologically tolerated salts for use in the treatment and/or prophylaxis of diseases of the bronchi.

10. Use of the compounds according to one of Claims 1, 2, 3, 4, 5 or 6 and/or their pharmacologically tolerated salts for the preparation of medicaments for the treatment and/or prophylaxis of diseases of the bronchi.

**Revendications**

1. Composés de formule I

(I)

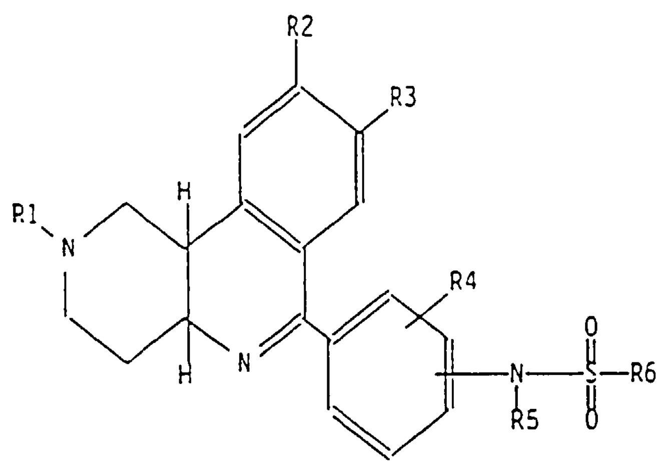

dans laquelle

$R^1$ représente un groupe alkyle en $C_{1-4}$,
$R^2$ représente un atome d'hydrogène (H), un groupe alkyle en $C_{1-4}$ ou alcoxy en $C_{1-4}$,

$R^3$ représente un atome d'hydrogène (H), un groupe alkyle en $C_{1-4}$ ou alcoxy en $C_{1-4}$,
$R^4$ représente un atome d'hydrogène (H), un groupe méthyle ou méthoxy,
$R^5$ représente un atome d'hydrogène (H) ou un groupe alkyle en $C_{1-4}$, et
$R^6$ représente un groupe alkyle en $C_{1-4}$, phényle ou phényle substitué portant un ou plusieurs substituants, identiques ou différents, choisis parmi les résidus alkyle en $C_{1-4}$, alcoxy en $C_{1-4}$, hydroxy et halogéno,

et les sels de tels composés.

**2.** Composés de formule I selon la revendication 1, dans lesquels

$R^1$ représente un groupe alkyle en $C_{1-4}$,
$R^2$ représente un groupe alcoxy en $C_{1-4}$,
$R^3$ représente un groupe alcoxy en $C_{1-4}$,
$R^4$ représente un atome d'hydrogène ou un groupe méthyle,
$R^5$ représente un atome d'hydrogène ou un groupe alkyle en $C_{1-4}$,
$R^6$ représente un groupe alkyle en $C_{1-4}$, un groupe phényle ou phényle substitué portant un substituant choisi parmi les résidus alkyle en $C_{1-4}$, alcoxy en $C_{1-4}$ et halogéno,

et les sels de tels composés.

**3.** Composés de formule I selon la revendication 1, dans lesquels

$R^1$ représente un groupe méthyle,
$R^2$ représente un groupe méthoxy,
$R^3$ représente un groupe méthoxy et
$R^4$ représente un atome d'hydrogène ou un groupe méthyle,

le résidu $-N(R^5)SO_2R^6$ est lié en position 4 au résidu phényle situé lui-même en position 6 du noyau benzo-naphtyridine,

$R^5$ représente un atome d'hydrogène, un groupe méthyle ou éthyle, et
$R^6$ représente un groupe méthyle, 4-méthylphényle, 4-méthoxyphényle ou 4-fluorophényle,

et les sels de tels composés.

**4.** rac-*cis*-8,9-diméthoxy-1,2,3,4,4a, 10b-hexahydrogéno-6-(4-méthylsulfonamidophényl)-2-méthylbenzo[c][1,6] naphtyridine et les sels de ce composé.

**5.** rac-*cis*-8,9-diméthoxy-1,2,3,4,4a,10b-hexahydrogéno-2-méthyl-6-[4-(p-toluènesulfonamido)-phényl]-benzo[c] [1,6]naphtyridine et les sels de ce composé.

**6.** (-)-*cis*-8,9-diméthoxy-1,2,3,4,4a,10b-hexahydrogéno-2-méthyl-6-[4-(p-toluènesulfonamido)-phényl]-benzo[c] [1,6]naphtyridine et les sels de ce composé.

**7.** Procédé de préparation de composés de formule I selon la revendication 1 et des sels de tels composés, caractérisé par le fait

a) que l'on fait réagir des composés de formule II

(II)

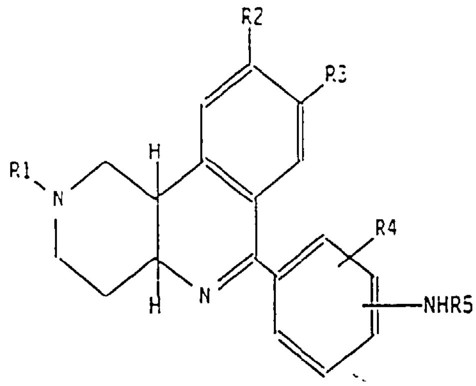

dans laquelle $R^1$, $R^2$, $R^3$, $R^4$ et $R^5$ ont la signification indiquée dans la revendication 1, avec des composés à groupe sulfonyle de formule (III)

(III)

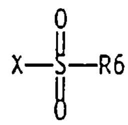

dans laquelle $R^6$ a la signification indiquée dans la revendication 1, et X représente un groupe partant approprié, ou

b) que l'on fait réagir des composés de formule (I) dans lesquels $R^5$ représente un atome d'hydrogène, avec un agent d'alkylation de formule (IV)

(IV) $Y\text{-}R^5$

dans laquelle $R^5$ représente un groupe alkyle en $C_{1-4}$ et Y un groupe partant, pour obtenir des composés de formule (I) dans lesquels $R^5$ représente un groupe alkyle en $C_{1-4}$, ou

c) que l'on soumet des composés de formule (V)

(V)

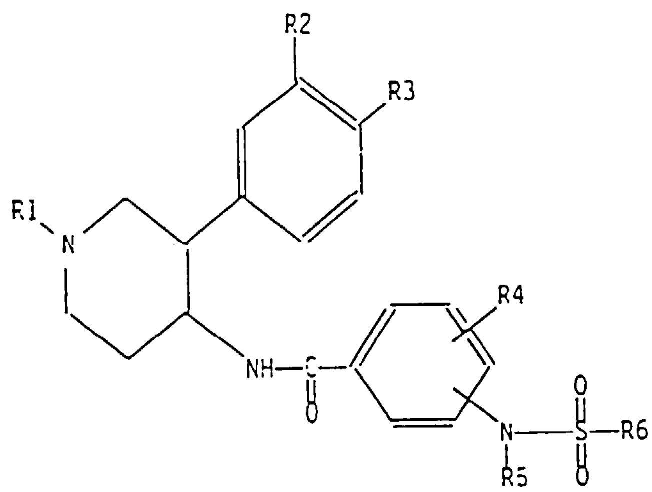

dans laquelle $R^1$, $R^2$, $R^3$, $R^4$, $R^5$ et $R^6$ ont les significations indiquées ci-dessus, à une condensation de cycle,

et en ce que l'on convertit éventuellement ensuite les composés de formule (I) obtenus selon les réactions a), b) ou c) en leurs sels, ou en ce que l'on libère éventuellement ensuite les composés de formule (I) à partir des sels des composés de formule (I).

8. Médicament contenant un ou plusieurs composés selon une des revendications 1, 2, 3, 4, 5 ou 6 et/ou les sels pharmacologiquement acceptables correspondants.

9. Composés selon une des revendications 1, 2, 3, 4, 5 ou 6 et/ou les sels pharmacologiquement acceptables correspondants, destinés à l'utilisation pour le traitement et/ou la prophylaxie de maladies des bronches.

10. Utilisation des composés selon une des revendications 1, 2, 3, 4, 5 ou 6 et/ou des sels pharmacologiquement acceptables correspondants pour la préparation de médicaments pour le traitement et/ou la prophylaxie de maladies des bronches.